# EUROPÄISCHE PATENTSCHRIFT

(11) **EP 2 862 502 B1**
(45) Veröffentlichungstag und Bekanntmachung des Hinweises auf die Patenterteilung: **02.06.2021**
(21) Anmeldenummer: 14187272.1
(22) Anmeldetag: 01.10.2014
(51) Int. Cl.: A61B 1/00, A61B 1/04

(54) **Endoskop**
Endoscope
Endoscope

(30) Priorität: 18.10.2013 DE 102013221224
(43) Veröffentlichungstag der Anmeldung: 22.04.2015
(73) Patentinhaber: Henke-Sass, Wolf GmbH, 78532 Tuttlingen (DE); Stryker Corporation, Kalamazoo, MI 49002 (US)
(72) Erfinder: Mattes, Andreas, 78589 Dürbheim (DE); Häckl, Norbert, 88637 Leibertingen (DE); Rapp, Stefan, 78050 Villingen-Schwenningen (DE); Weller, Thomas, 78532 Tuttlingen (DE); Lawrence, Hannah, San Jose, CA 95122 (US); Pack,Candice, Campbell, CA 95008 (US)
(74) Vertreter: Patentanwälte Geyer, Fehners & Partner mbB

(56) Entgegenhaltungen:
- EP-A1- 0 489 240
- WO-A1-99/39623
- US-A- 5 609 561

## Beschreibung

Die vorliegende Erfindung betrifft ein Endoskop mit einem Endoskopschaft und einem mit dem Endoskopschaft verbundenen Hauptkörper, der einen Kameraanschluß aufweist, an dem eine optische Aufnahmevorrichtung mechanisch befestigbar ist, wobei der Hauptkörper elektrisch leitfähig ist.

Die EP 0 489 240 A1 zeigt ein Endoskop mit den Merkmalen des Oberbegriffs des Anspruchs 1. Dabei ist die mechanische Befestigbarkeit der optischen Aufnahmevorrichtung an dem Kameraanschluß durch eine Rastmechanik bereitgestellt, die mechanisch sehr aufwendig ist und aufgrund der vorzusehenden Feder mit der Zeit schlechtere mechanische Verbindungseigenschaften aufweist.

Die US 5,609,561 zeigt ein Endoskop, bei dem eine mit dem Hauptkörper des Endoskops verbindbare Kamera einen Linsen aufweisenden Adapter aufweist, dessen Gehäuse aus einem autoklavierbaren Material, wie z.B. einem metallischen Material oder einem geeigneten Kunststoffmaterial, gebildet ist.

Bei dem Einsatz eines solchen Endoskopes zur optischen Kontrolle bei der Hochfrequenzchirurgie kann in unerwünschter Weise die Schwierigkeit auftreten, dass dabei erzeugte Spannungspulse über das Endoskop bis zu der am Endoskop befestigten optischen Aufnahmevorrichtung übertragen werden. Diese Spannungspulse können zu einer Verschlechterung der Aufnahme des Bildes mit dem Bildsensor der optischen Aufnahmevorrichtung führen. Bei CCD-Sensoren kann dies insbesondere zu einem verrauschten Bild führen. Wenn CMOS-Sensoren eingesetzt werden, kann insbesondere aufgrund der digitalen Signalverarbeitung sogar ein totaler Bildausfall auftreten.

Erfindungsgemäß ist es daher Aufgabe der vorliegenden Erfindung, das eingangs genannte Endoskop so zu verbessern, dass die genannten Schwierigkeiten möglichst vollständig überwunden werden können.

Die Erfindung ist im Anspruch 1 definiert. Vorteilhafte Weiterbildungen sind in den abhängigen Ansprüchen angegeben.

Damit wird sichergestellt, dass eine gute Verbindung zwischen dem Kameraanschluß und der optischen Aufnahmevorrichtung vorliegt und dass diese Verbindung mehrfach gelöst und wieder hergestellt werden kann, ohne dass es zu einer Verschlechterung der mechanischen Verbindungseigenschaften kommt.

Da der Kameraaanschluß den elektrischen Isolierkörper aufweist, der eine am Kameraanschluß mechanisch befestigte optische Aufnahmevorrichtung vom Hauptkörper elektrisch isoliert, wird eine elektrische Isolierung erzeugt, die dazu führt, dass die unerwünschten Spannungspulse vom Endoskop nicht mehr bis zur optischen Aufnahmevorrichtung übertragen werden, so dass die optische Aufnahmevorrichtung unbeeinflußt von eventuell auftretenden Spannungspulsen in gewünschter Weise das Bild aufnehmen kann.

Bei dem erfindungsgemäßen Endoskop ist somit im Vergleich zu bekannten Endoskopen der Kameraanschluß so abgewandelt, dass er den elektrischen Isolierkörper aufweist. Damit können in einfacher Art und Weise auch schon vorhandene Endoskope so umgerüstet werden, dass sie als erfindungsgemäße Endoskope die gewünschte elektrische Isolierung zwischen der optischen Aufnahmevorrichtung und dem restlichen Endoskop aufweisen.

Insbesondere kann der Kameraanschluß am proximalen Ende des Endoskopes angeordnet sein. Des weiteren kann der Kameraanschluß gegenüber dem Hauptkörper drehbar sein. Insbesondere kann der Kameraanschluß um eine Längsachse des Endoskopes drehbar sein.

Insbesondere kann der elektrische Isolierkörper zumindest einen gegenüber dem Hauptkörper drehbaren Ring aufweisen und kann der Gewindering drehfest mit dem zumindest einen Ring verbunden sein.

Damit wird einerseits die Drehbarkeit des Kameraanschlusses bereitgestellt und andererseits die notwendige elektrische Isolierung sichergestellt.

Der elektrische Isolierkörper kann aus einem Kunststoffmaterial gebildet sein. Insbesondere kann z.B. Polyetheretherketon (PEEK) verwendet werden. Dieser Kunststoff zeichnet sich durch seine hohe Schmelztemperatur aus und kann wiederholt sterilisiert bzw. autoklaviert werden.

Der elektrische Isolierkörper kann natürlich auch aus jedem anderen geeigneten dielektrischen Material hergestellt sein. Wesentlich ist, dass das Material die gewünschte elektrische Isoliereigenschaft aufweist.

Der elektrische Isolierkörper kann einteilig oder mehrteilig ausgebildet sein.

Das erfindungsgemäße Endoskop ist bevorzugt als optisches Endoskop ausgebildet, das ein vor dem Endoskopschaft befindliches Objekt als Bild in eine Fokusebene abbildet, die hinter dem vom Endoskopschaft abgewandten Ende des Hauptkörpers (und somit außerhalb des Hauptkörpers) liegt. Dazu kann im Endoskopschaft und im Hauptkörper eine Abbildungsoptik angeordnet sein.

Insbesondere kann die Abbildungsoptik in einem hermetisch dichten Innenraum des Endoskopes angeordnet sein.

Bevorzugt ist das Endoskop autoklavierbar. Unter autoklavierbar wird hier insbesondere verstanden, dass das Endoskop während einer vorbestimmten Zeitdauer (beispielsweise mehrere Minuten) Wasserdampf (insbesondere gesättigtem Wasserdampf) von mindestens 100°C bzw. mindestens 130°C zur Sterilisation ausgesetzt wird, ohne dass dabei das Endoskop beschädigt wird (ohne dass insbesondere Wasserdampf in den Endoskopschaft oder den Innenraum des Hauptkörpers eindringen kann).

Der Endoskopschaft und der Hauptkörper sind bevorzugt im wesentlichen aus einem metallischen Material, wie z.B. Edelstahl, hergestellt. Ferner sind der Endoskopschaft und der Hauptkörper bevorzugt elektrisch leitfähig und auch elektrisch leitfähig miteinander verbunden.

Der Endoskopschaft kann als starrer Endoskopschaft, als Endoskopschaft mit einem abwinkelbaren distalen Ende oder als flexibler Endoskopschaft ausgebildet sein.

Das erfindungsgemäße Endoskop kann als medizinisches Endoskop oder als technisches Endoskop ausgebildet sein.

Die optische Aufnahmevorrichtung kann insbesondere eine digitale Kamera bzw. eine digitale Videokamera sein.

Die optische Aufnahmevorrichtung kann, muß aber nicht, Bestandteil des erfindungsgemäßen Endoskopes sein.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer Ausführungsform des erfindungsgemäßen Endoskopes;
- Fig. 2: eine Schnittansicht des Endoskopes von Fig. 1, und
- Fig. 3: eine vergrößerte Schnittansicht des proximalen Endbereichs des Hauptkörpers 4 des Endoskops von Fig.1 und 2.

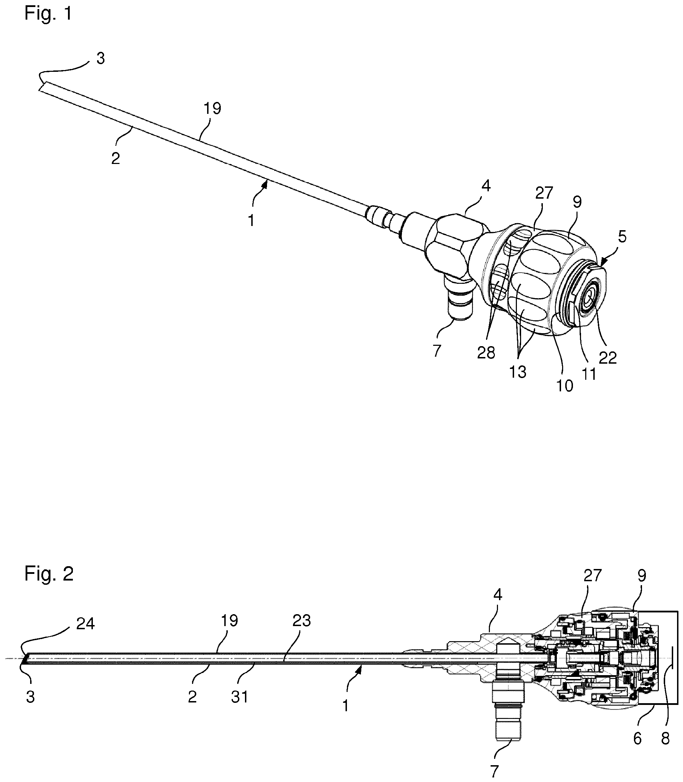

Bei der in Fig. 1 und 2 gezeigten Ausführungsform umfaßt das erfindungsgemäße Endoskop 1 einen Endoskopschaft 2 mit einem distalen Ende 3, das gleichzeitig das distale Ende des Endoskops 1 ist. Das dem distalen Ende 3 abgewandte Ende des Endoskopschaftes 2 ist mit einem Hauptkörper 4 des Endoskops verbunden, der einen Kameraanschluß 5 (z.B. ein C-Mount-Anschluß oder ein Bajonettanschluß) für eine optische Aufnahmevorrichtung 6 (beispielsweise eine Videokamera) sowie einen Beleuchtungslichtanschluß 7 aufweist. Die optische Aufnahmevorrichtung 6, die beispielsweise einen CMOS-Sensor 8 enthält, ist lediglich in der Darstellung von Fig. 2 schematisch eingezeichnet und kann, muß aber nicht, Teil des erfindungsgemäßen Endoskops 1 sein.

Der Endoskopschaft 2 und der Hauptkörper 4 sind in der Regel zum überwiegenden Teil aus Metall (z.B. Edelstahl) hergestellt und somit elektrisch leitfähig.

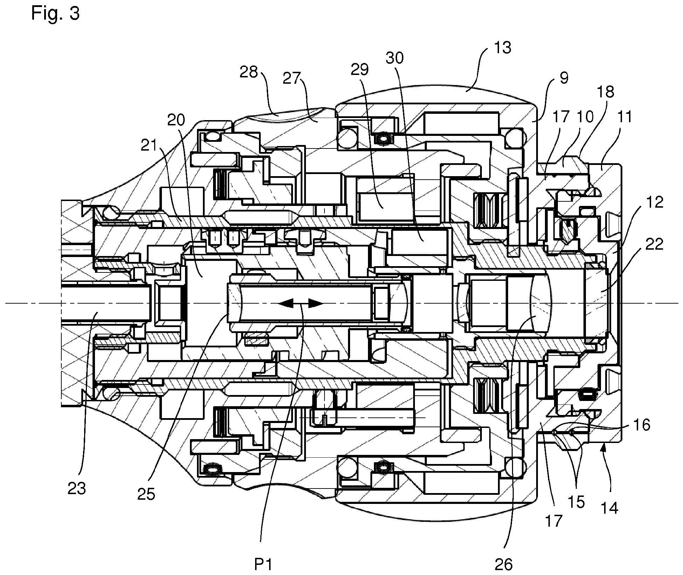

Wie insbesondere in der vergrößerten Schnittdarstellung von Fig. 3 ersichtlich ist, umfaßt der Kameraanschluß 5 einen Montagering 9, einen metallischen Gewindering 10, einen Fixierring 11 und einen Innenring 12, wobei der Montagering 9, der Fixierring 11 und der Innenring 12 aus einem elektrisch nicht leitfähigen Material und somit insbesondere aus einem elektrisch isolierenden Material gebildet sind. Als Material kann ein Kunststoffmaterial verwendet werden, wie z.B. PEEK (Polyetheretherketon), das autoklavierbar ist.

Der Montagering 9, der Gewindering 10 und der Fixierring 11 sind drehfest miteinander verbunden und können zusammen um die Längsachse des Endoskopes 1 relativ zu den restlichen Teilen des Hauptkörpers 4 sowie relativ zum Innenring 11 gedreht werden. Der Gewindering 10 weist ein Außengewinde 18 auf, auf das ein entsprechendes Innengewinde der optischen Aufnahmevorrichtung 6 aufgeschraubt werden kann. Um dieses Aufschrauben zu erleichtern, ist die beschriebene Drehbarkeit von Montagering 9, Gewindering 10 und Fixierring 11 vorgesehen, so dass durch Drehung dieser drei Ringe 9 bis 11 ein Aufschrauben der optischen Aufnahmevorrichtung 6 auf den Kameraanschluß 5 verwirklicht werden kann. Um dieses Aufschrauben zu erleichtern, weist der Montagering 9 Griffmulden 13 auf, die in Umfangsrichtung nebeneinanderliegend angeordnet sind.

Da der Montagering 9, der Fixierring 11 und der Innenring 12 aus einem elektrisch isolierendem Material gebildet sind, bilden sie zusammen einen elektrischen Isolierkörper 14, der dafür sorgt, dass die mit dem Kameraanschluß 5 mechanisch verbundene optische Aufnahmevorrichtung 6 vom Hauptkörper 4 elektrisch isoliert ist. Es liegt im Bereich des Kameraanschlusses 5 keine elektrische Verbindung zwischen dem Hauptkörper 4 und der optischen Aufnahmevorrichtung 6 vor.

Damit wird der Vorteil erreicht, dass Spannungspulse, die z.B. bei der Hochfrequenzchirurgie oder dem Einsatz eines Elektroskalpells auftreten können und in unerwünschter Weise auf das zur optischen Kontrolle vorgesehene Endoskop 1 und somit auf den Endoskopschaft 2 und von diesem zum Hauptkörper 4 übertragen werden können, nicht zur optischen Aufnahmevorrichtung 6 übertragen werden, da diese aufgrund des elektrischen Isolierkörpers 14 elektrisch vom Hauptkörper 4 isoliert ist. Solche Spannungspulse würden gerade bei CMOS-Sensoren 8 dazu führen, dass ein unerwünschter Bildausfall auftritt. Dies kann bei dem erfindungsgemäßen Endoskop 1 aufgrund des elektrischen Isolierkörpers 14 effektiv verhindert werden.

Die Ausbildung des Gewinderings 10 als metallischer Gewindering 10 führt zu dem Vorteil, dass einerseits eine gute mechanische Verbindung mit der optischen Aufnahmevorrichtung 6 möglich ist. Andererseits ist ein wiederholtes Auf- und Abschrauben möglich, ohne dass es zu einer Verschlechterung der mechanischen Verbindung kommen würde. Nachdem der metallische Gewindering 10 nur mit dem Montagering 9 und dem Fixierring 11, die beide elektrisch nicht leitend sind, in Kontakt steht, ist somit der metallische Gewindering 10 ebenfalls durch den elektrischen Isolierkörper 14 vom Hauptkörper 4 elektrisch isoliert.

Wie insbesondere Fig. 3 zu entnehmen ist, weist der Gewindering 10 auf seiner Innenseite mehrere nach innen vorstehende Nasen 15 auf, die in Formschluß mit entsprechenden nutförmigen Ausnehmungen 16 auf der Außenseite eines Trageabschnitts 17 des Montagerings 9 stehen, so dass eine drehfeste Verbindung zwischen dem Gewindering 10 und dem Montagering 9 vorliegt. Somit kann der Gewindering 10 nicht relativ zum Montagering 9 um die Längsachse des Endoskops gedreht werden.

Das Endoskop 1 ist autoklavierbar ausgebildet. Dazu ist ein hermetisch dichter Innenraum 20 vorgesehen, der im proximalen Endbereich des Hauptkörpers 4, wie in Fig. 3 gezeigt ist, durch eine Wandung 21 begrenzt ist und am proximalen Ende durch ein proximales Deckglas 22 hermetisch dicht verschlossen ist. Der Innenraum 20 ist mit einem Innenrohr 23 des Endoskopschaftes 2 verbunden, das sich in einem Außenrohr 19 des Endoskopschaftes 2 bis zum distalen Ende 3 erstreckt. Am distalen Ende 3 ist das Innenrohr 23 mit einem distalen Deckglas 24 (Fig. 2) hermetisch dicht verschlossen.

Im Innenrohr 23 ist hinter dem distalen Deckglas 24 ein (nicht gezeigtes) Objektiv zur Aufnahme eines Bildes eines vor dem distalen Ende 3 befindlichen Objektes angeordnet. An das Objektiv kann sich ein bekanntes Lichtleitsystem (z.B. Stablinsen oder Umkehrsysteme, die ein eingangsseitiges Bild ausgangsseitig um 180° gedreht abbilden) anschließen, das das Bild bis zu dem proximalen Endbereich im Hauptkörper 4 überträgt, in dem einerseits eine in Längsrichtung des Endoskopes 1 verschiebbare Optikgruppe 25 (wie durch den Doppelpfeil P1 angedeutet ist) und andererseits eine fixe Optikgruppe 26, die somit nicht in Längsrichtung des Endoskopes 1 verschiebbar ist, angeordnet ist (beide Optikgruppen 25, 26 sind im Innenraum 20 angeordnet). Damit ist beispielsweise eine Fokussierung auf unterschiedliche Abstände der Bildsensoren 8 unterschiedlicher optischer Aufnahmevorrichtung 6 möglich. Die Verschiebung der verschiebbaren Optikgruppe 25 kann in bekannter Weise realisiert sein. So kann beispielsweise ein Fokussierring 27 mit in Umfangsrichtung voneinander beabstandeten Griffmulden 28 vorgesehen sein, der zumindest einen Außenmagnet 29 trägt. Innerhalb des Innenraums 20 ist zumindest ein Innenmagnet 30 vorgesehen, der drehbar gelagert ist, so dass eine Drehung des Fokussierringes 27 über die Magnetkopplung der beiden Magnete 29, 30 zu einer Drehung der Lagerung des Innenmagnetes 30 dient. Diese Drehung wird in eine axiale Bewegung der verschiebbaren Optikgruppe 25 umgesetzt.

In einer alternativen Ausbildung des erfindungsgemäßen Endoskopes 1 ist keine verschiebbare Optikgruppe 25 und ggf. keine fixe Optikgruppe 26 vorgesehen.

Ferner kann das Endoskop, wie in Fig. 2 angedeutet ist, zwischen dem Innenrohr 23 und dem Außenrohr 19 des Endoskopschaftes 2 einen Kanal 31 aufweisen, der sich vom Beleuchtungslichtanschluß 7 bis zum distalen Ende 3 erstreckt und in dem Lichtleitfasern (nicht gezeigt) angeordnet sind, die über den Beleuchtungslichtanschluß 7 mit Licht beaufschlagt werden, so dass dieses Licht am distalen Ende 3 des Endoskops austritt und zur Beleuchtung des vor dem distalen Ende 3 aufzunehmenden Objektes verwendet werden kann.

## Patentansprüche

1. Endoskop mit
einem Endoskopschaft (2) und
einem mit dem Endoskopschaft (2) verbundenen Hauptkörper (4), der einen Kameraanschluß (5) aufweist, an dem eine optische Aufnahmevorrichtung (6) mechanisch befestigbar ist,
wobei der Hauptkörper (4) elektrisch leitfähig ist und
der Kameraanschluß (5) einen elektrischen Isolierkörper (14) aufweist,
**dadurch gekennzeichnet, dass** der Kameraanschluß (5) einen metallischen Gewindering (10) mit einem Außengewinde (18) aufweist, auf das ein entsprechendes Innengewinde der optischen Aufnahmevorrichtung (6) aufschraubbar ist,
wobei der metallische Gewindering (10) so auf dem elektrischen Isolierkörper (14) angeordnet ist, dass der metallische Gewindering (10) vom Hauptkörper (4) elektrisch isoliert ist, um eine am Kameraanschluß (5) mechanisch befestigte optische Aufnahmevorrichtung (6) vom Hauptkörper (4) elektrisch zu isolieren.

2. Endoskop nach Anspruch 1, bei dem der Kameraanschluß (5) am proximalen Ende des Endoskopes (1) angeordnet ist.

3. Endoskop nach Anspruch 1 oder 2, **dadurch gekennzeichnet, dass** der Kameraanschluß (5) gegenüber dem Hauptkörper (4) drehbar ist.

4. Endoskop nach einem der obigen Ansprüche, **dadurch gekennzeichnet, dass** der elektrische Isolierkörper (14) zumindest einen gegenüber dem Hauptkörper (4) drehbaren Ring (9, 11) aufweist und der Gewindering (10) drehfest mit dem zumindest einen Ring (9, 11) verbunden ist.

5. Endoskop nach einem der obigen Ansprüche, **dadurch gekennzeichnet, dass** der elektrische Isolierkörper (14) aus einem Kunststoffmaterial gebildet ist.

6. Endoskop nach einem der obigen Ansprüche, **dadurch gekennzeichnet, dass** der elektrische Isolierkörper (14) aus Polyetheretherketon hergestellt ist.

7. Endoskop nach einem der obigen Ansprüche, **dadurch gekennzeichnet, dass** im Endoskopschaft (2) und im Hauptkörper (4) eine Abbildungsoptik (25, 26) angeordnet ist, die ein vor dem Endoskopschaft (2) befindliches Objekt als Bild in eine Fokusebene abbildet, die hinter dem vom Endoskopschaft (2) abgewandten Ende des Hauptkörpers (4) liegt.

8. Endoskop nach Anspruch 7, **dadurch gekennzeichnet, dass** die Abbildungsoptik (25, 26) in einem hermetisch dichten Innenraum (20) des Endoskopes (1) angeordnet ist.

9. Endoskop nach einem der obigen Ansprüche, **dadurch gekennzeichnet, dass** das Endoskop autoklavierbar ist.

## Claims

1. An endoscope with
an endoscope shaft (2) and
a main body (4) connected to the endoscope shaft (2), which body comprises a camera connection (5) to which an optical recording device (6) can be mechanically fixed,
wherein the main body (4) is electrically conductive and
the camera connection (5) comprises an electric insulating body (14),
**characterized in that** the camera connection (5) comprises a metallic ring nut (10) with an external thread (18) onto which a corresponding internal thread of the optical recording device (6) can be screwed,
wherein the metallic ring nut (10) is arranged on the electric insulating body (14) such that the metallic ring nut (10) is electrically insulated from the main body (4) in order to insulate an optical recording device (6), mechanically fixed to the camera connection (5), from the main body (4).

2. The endoscope according to claim 1, in which the camera connection (5) is arranged at the proximal end of the endoscope (1).

3. The endoscope according to claim 1 or 2, **characterized in that** the camera connection (5) can be rotated relative to the main body (4).

4. The endoscope according to any of the above claims, **characterized in that** the electric insulating body (14) comprises at least one ring (9, 11) which can be rotated relative to the main body (4) and the ring nut (10) is connected to the at least one ring (9, 11) in rotation-resistant manner.

5. The endoscope according to any of the above claims, **characterized in that** the electric insulating body (14) is formed from a plastic material.

6. The endoscope according to any of the above claims, **characterized in that** the electric insulating body (14) is produced from polyetheretherketone.

7. The endoscope according to any of the above claims, **characterized in that** imaging optics (25, 26) are arranged in the endoscope shaft (2) and in the main body (4), which imaging optics display an object located in front of the endoscope shaft (2) as an image in a focussing plane which lies behind the end of the main body (4) facing away from the endoscope shaft (2).

8. The endoscope according to claim 7, **characterized in that** the imaging optics (25, 26) are arranged in a hermetically sealed inner space (20) of the endoscope (1).

9. The endoscope according to any of the above claims, wherein the endoscope is capable of being autoclaved.

## Revendications

1. Endoscope comprenant
une tige d'endoscope (2) et
un corps principal (4) qui est relié à la tige d'endoscope (2) et qui comporte un raccord de caméra (5) auquel un dispositif d'enregistrement optique (6) peut être fixé mécaniquement, le corps principal (4) étant électriquement conducteur et le raccord de caméra (5) comportant un corps électriquement isolant (14), **caractérisé en ce que** le raccord de caméra (5) comporte une bague filetée métallique (10) pourvue d'un filetage externe (18) sur lequel un filetage interne correspondant du dispositif d'enregistrement optique (6) peut être vissé,
la bague filetée métallique (10) étant disposée sur le corps isolant électrique (14) de telle sorte que la bague filetée métallique (10) soit isolée électriquement du corps principal (4) afin d'isoler électriquement un dispositif d'enregistrement optique (6), fixé mécaniquement au raccord de caméra (5), du corps principal (4).

2. Endoscope selon la revendication 1, dans lequel le raccord de caméra (5) est disposée à l'extrémité proximale de l'endoscope (1).

3. Endoscope selon la revendication 1 ou 2, **caractérisé en ce que** le raccord de caméra (5) peut tourner par rapport au corps principal (4).

4. Endoscope selon l'une des revendications précédentes, **caractérisé en ce que** le corps isolant électrique (14) comporte au moins une bague (9, 11) pouvant tourner par rapport au corps principal (4) et la bague filetée (10) est reliée solidairement en rotation à l'au moins une bague (9, 11).

5. Endoscope selon l'une des revendications précédentes, **caractérisé en ce que** le corps isolant électrique (14) est formé à partir d'une matière synthétique.

6. Endoscope selon l'une des revendications précédentes, **caractérisé en ce que** le corps isolant électrique (14) est en polyétheréthercétone.

7. Endoscope selon l'une des revendications précédentes, **caractérisé en ce qu'**une optique de reproduction (25, 26) est disposée dans la tige d'endoscope (2) et dans le corps principal (4) et reproduit un objet situé devant la tige d'endoscope (2) sous la forme d'une image dans un plan focal qui est situé derrière l'extrémité du corps principal (4) qui est opposée à la tige d'endoscope (2).

8. Endoscope selon la revendication 7, **caractérisé en ce que** l'optique de reproduction (25, 26) est disposée dans un espace intérieur hermétique (20) de l'endoscope (1) .

9. Endoscope selon l'une des revendications précédentes, **caractérisé en ce que** l'endoscope peut être passé à l'autoclave.
